# NEUE EUROPÄISCHE PATENTSCHRIFT

(11) **EP 0 581 044 B2**
(45) Veröffentlichungstag und Bekanntmachung des Hinweises auf die Entscheidung über den Einspruch: **02.01.2004**
(45) Hinweis auf die Patenterteilung: 20.05.1998
(21) Anmeldenummer: 93110555.5
(22) Anmeldetag: 30.06.1993
(51) Int. Cl.: A61F 13/15

(54) **Wegwerfwindel**
Disposable diaper
Couche à jeter

(30) Priorität: 01.07.1992 JP 4591492
(43) Veröffentlichungstag der Anmeldung: 02.02.1994
(73) Patentinhaber: UNI-CHARM CORPORATION, Kawanoe-shi Ehime-ken (JP)
(72) Erfinder: Igaue, Takamitsu, Kawanoe-shi, Ehime-ken (JP); Inoue, Kohji, Kanonji-shi, Kagawa-ken (JP); Kido, Tsutomu, Kawanoe-shi, Ehime-ken (JP)
(74) Vertreter: Sperling, Rüdiger, Dipl.-Ing.

(56) Entgegenhaltungen:
- EP-A- 0 357 298
- EP-A- 0 359 410
- EP-A- 0 486 006
- FR-A- 2 495 899
- FR-A- 2 561 078
- GB-A- 2 242 821
- US-A- 4 834 737
- US-A- 5 037 413
- US-A- 5 171 236
- US-A- 5 207 663

## Beschreibung

Die Erfindung betrifft Wegwerfwindeln, die ausgelegt sind, Körperausscheidungen wie Urin und Exkremente aufzusaugen und zu halten. Eine Solche Windel ist bekannt aus dem Dokument EP-A-0 359 410. Diese Entgegen haltung entspricht den Oberbegriff des Anspruchs 1.

In der japanischen Patentanmeldung Nr. 1991-231664 ist eine Wegwerfwindel veröffentlicht, die eine erste flüssigkeitsdurchlässige obere Lage, eine flüssigkeitsundurchlässige untere Lage, einen flüssigkeitsabsorbierenden Körper, der zwischen den genannten Lagen sachwichartig eingelegt ist und eine zweite flüssigkeitsdurchlässige obere Lage, die die erste flüssigkeitsdurchlässige Lage überdeckt und an in Längsrichtung einander gegenüberliegendenen Enden dieser ersten Lage befestigt ist. Mit der in der oben genannten Patentanmeldung offenbarten Windel wird angestrebt, die zweite flüssigkeitsdurchlässige Lage in Kontakt mit der Haut des Trägers im Schrittbereich in Kontakt zu bringen, um die Exkremente sicher zurückzuhalten. Abhängig von der besonderen Beschaffenheit der Ausscheidungen oder Exkremente, wie auch abhängig von der Paßform der zweiten flüssigkeitsdurchlässigen durchlässigen oberen Lage im Schrittbereich, ist es jedoch zu befürchten, daß Exkremente zwischen der Haut des Trägers und der flüssigkeitsdurchlässigen zweiten oberen Lage hindurchgedrückt und somit die Haut des Trägers über einen weiten Bereich mit in solcher Weise austretenden Exkrementen beschmutzt werden. Diese Exkremente haften an den Geschlechts- und Ausscheidungsorganen des Trägers, so daß ernsthafte Probleme bezüglich der Hygiene entstehen können.

Es ist daher eine wesentliche Aufgabe der Erfindung, die zweite flüssigkeitsdurchlässige obere Lage so zu verbessern, daß die oben erwähnten Probleme wirkungsvoll überwunden werden können, selbst wenn diese zweite flüssigkeitsdurchlässige obere Lage zur Anwendung gelangt.

### ZUSAMMENFASSUNG DER ERFINDUNG.

Die oben erwähnte Aufgabe wird erfindungsgemäß durch eine Wegwerfwindel gemäß den Merkmalen das Anspruchs 1 gelöst.

Während die zweite flüssigkeitsdurchlässige obere Lage über dem Schrittbereich mit der Haut des Trägers in Kontakt tritt, wird jeweils eine der Öffnungen sauber mit dem Urinalorgan und dem Anus zur Deckung gebracht, so daß austretende Ausscheidungen durch die jeweiligen Öffnungen in einen Raum fliessen können, der zwischen der zweiten flüssigkeitsdurchlässigen oberen Lage und der ersten flüssigkeitsdurchlässigen Lage, welche unter der zweiten flüssigkeitsdurchlässigen Lage liegt, gebildet ist.

Andere besondere Ausführungen gemäß der Erfindung sind in den Ansprüchen 2-4 angegeben.

### KURZBESCHREIBUNG DER ZEICHNUNGEN.

Die Erfindung wird nachfolgend unter Zugrundelegung eines Ausführungsbeispiels unter Bezugnahme auf die beiliegenden Zeichnungen näher im Detail erläutert. In den Figuren zeigen:
- Fig. 1: eine perspektivische Darstellung einer erfindungsgemäßen Windel im aufgestellten Zustand,
- Fig. 2: eine Ansicht auf eine Abwicklung der Windel mit teilweise herausgebrochenen Bereichen, und
- Fig. 3: eine Ansicht eines Schnitts entlang der Linie X-X in Fig. 2, wobei die Windel im gekrümmten Zustand dargestellt ist.

### BESCHREIBUNG EINES BEVORZUGTEN AUSFÜHRUNGSBEISPIELS.

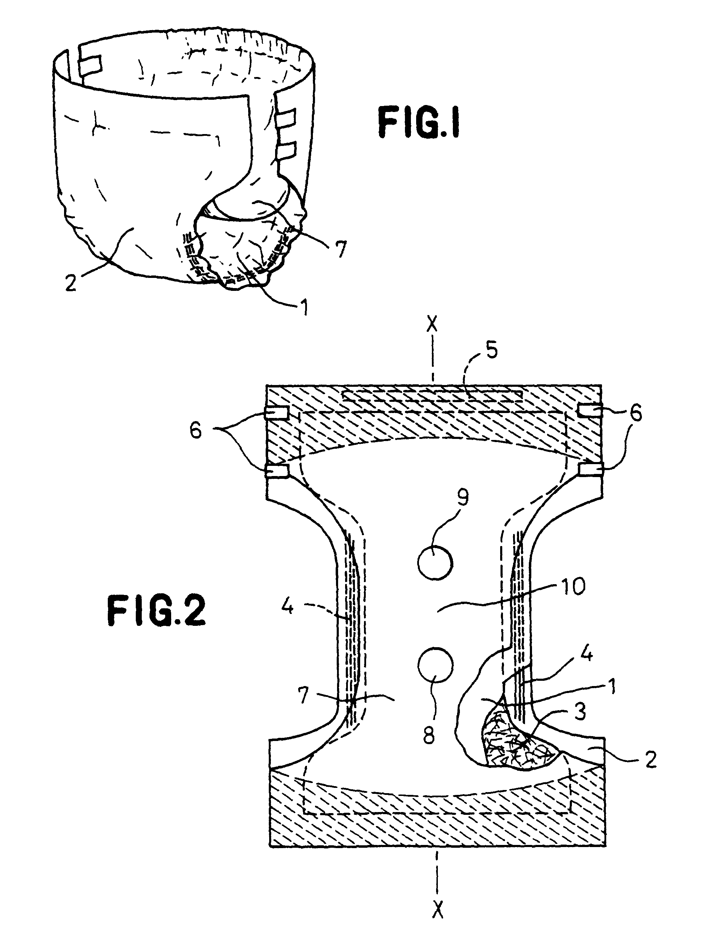

Es wird bezug genommen auf die Figuren 1 und 2. Die darin dargestellte Windel weist eine Windelgrundstruktur mit einer ersten flüssigkeitsdurchlässigen oberen Lage 1, einer flüssigkeitsundurchlässigen unteren Lage 2, einen flüssigkeitsabsorbierenden Körper 3, der zwischen der oberen und der unteren Lage 1 bzw. 2 sandwichartig eingelegt ist, elastische Teile 4 und 5, die zwischen den oberen und der unteren Lage 1 bzw. 2 entlang seitlich einander gegenüberliegender Seiten und entlang der rückwärtigen Körpertaille angeordnet und Befestiger 6 aufweist, die an seitlich einander gegenüberliegenden Enden der hinteren Körpertaille befestigt sind.

Eine zweite flüssigkeitsdurchlässige obere Lage 7 ist über der ersten flüssigkeitsdurchlässigen oberen Lage 1 angeordnet und an den in Längsrichtung einander gegenüberliegenden Enden an der ersten flüssigkeitsdurchlässigen oberen Lage 1 und der flüssigkeitsundurchlässigen unteren Lage 2 befestigt. Dies ist in Fig. 2 mit durch gestrichelte Linien schraffierte Bereiche angezeigt. Die zweite obere Lage 7 ist sowohl in Längsals auch in Querrichtung dehnbar und wird in einem Zustand, in dem sie sowohl in Quer- als auch in Längsrichtung gedehnt ist, mit ihren Längsenden an der Windelgrundstruktur befestigt, so daß die zweite obere Lage 7 eine aktuelle Breite aufweist, die kleiner ist als die Breite der ersten oberen Lage 1. Die zweite obere Lage 7 ist an den Stellen, die den Urinalorganen und dem Anus des Trägers entsprechen, wenn die Windel am Träger angelegt ist, mit Öffnungen 8,9 versehen, die so dimensioniert sind, daß Urin und Exkremente, die von den jeweiligen Organen ausgeschieden werden, durch die jeweiligen Öffnungen 8,9 hindurchtreten, ohne daß die Oberfläche der zweiten Lage 7 beschmutzt wird. Diese Öffnungen 8,9 sind unabhängig voneinander und weisen zwischen sich einen Bereich 10 auf. Obwohl in der Zeichnung nicht dargestellt, kann ein elastisches Teil zwischen den Öffnungen 8 und 9 am seitlichen Zentrum des Abschnitts 10 vorgesehen sein.

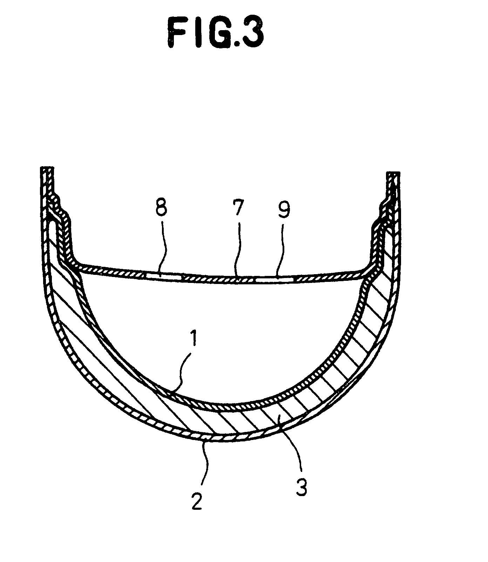

Die zweite obere Lage 7 weist eine Längsspannung auf, die bei 20 bis 197 g/cm (50 bis 500 g/inch) liegt. Der Saugkörper 3 besitzt eine Biegefestigkeit, die zwischen 5 und 20g ·cm eingestellt ist, gemessen nach der Taber-Methode. Dies hat zur Folge, daß die erste und die zweite obere Lage 1 und 7 einen Abstand voneinander, bei einem Maximum vorzugsweise von 10 mm oder mehr aufweisen, wenn die Windel unter der Längsspannung der zweiten oberen Lage 7, wie in Fig. 3 dargestellt, gekrümmt wird.

Die oberen Lagen 1,7 können aus beliebigem Faservlies hergestellt sein. Die untere Lage 2 kann aus jedem geeigneten Kunststoffilm bestehen und schließlich kann der Saugkörper 3 aus beliebiger geeignet bauschiger Pulpe, gemischt mit superabsorbierendem Polymer bestehen.

Bei einer erfindungsgemäßen Wegwerfwindel, die in der oben beschriebenen Weise mit einer zweiten flüssigkeitsdurchlässigen oberen Lage, welche ein Paar Öffnungen, 8,9 aufweist, die jeweils gegenüber den Auscheidungsorganen angeordnet sind, wenn die zweite flüssigkeitsdurchlässige obere Lage 7 die Haut des Trägers im Schrittbereich berührt, fließen ausgeschiedener Urin und Exkremente von den entsprechenden Organen durch die jeweiligen Öffnungen 8,9 unmittelbar in den Raum, der Zwischen der zweiten flüssigkeitsdurchlässigen oberen Lage 7 und der ersten flüssigkeitsdurchlässigen Lage 1 gebildetwird. Dementsprechend ist nicht zu befürchten, daß die Haut des Trägers mit Exkrementen beschmutzt wird, die zwischen der Haut des Trägers und der zweiten flüssigkeitsdurchlässigen oberen Lage 7 hindurchgequetscht und verteilt würden. Insbesondere wirkt der Abschnitt 10 der zweiten flüssigkeitsdurchlässigen oberen Lage 7 zwischen den Öffnungen 8,9, welche in enger Berührung mit der Haut des Trägers steht, als eine Barriere, um zu verhindern, daß die Exkremente ausfließen und die Genitalund anderen Ausscheidungsorgane beschmutzen.

## Patentansprüche

1. Wegwerfwindel mit einer ersten flüssigkeistdurchlässigen oberen Lage (1), einer flüssigkeitsundurchlässigen unteren Lage (2) und einem flüssigkeitsabsorbierenden Körper (3), der sandwichartig zwischen den genannten Lagen eingelegt ist, wobei diese Lagen eine Windelgrundstruktur bilden, und mit einer zusätzlichen flüssigkeitsdurchlässigen oberen Lage (7), die über die erste flüssigkeitsdurchlässige obere Lage (1) gelegt ist und an ihren in Längsrichtung einander gegenüberliegenden Enden an der genannten Windelstruktur befestigt ist und in der eine Öffnung ausgebildet ist, wobei die zweite flüssigkeitsdurchlässige obere Lage (7) aus einer sowohl in Längsrichtung als auch in Querrichtung dehnbaren Lage (7) besteht und in in Längsrichtung gedehntem Zustand an der genannten Windelstruktur befestigt ist,
**dadurch gekennzeichnet,**
**daß** die zweite flüssigkeitsdurchlässige Lage (7) auch in in Querrichtung gedehntem Zustand nur an den genannten Stellen befestigt ist und an Stellen, die den Urinalorganen und dem Anus eines Trägers entsprechen, mit voneinander unabhängigen Öffnungen versehen ist.

2. Wegwerfwindel nach Anspruch 1, wobei die zweite obere Lage eine Dehnung aufweist, die bei 50 bis 500 g/Zoll eingestellt ist und der Saugkörper (3) eine Biegefestigkeit aufweist, die bei 5 bis 20 g·cm eingestellt ist, gemessen nach der Taber-Methode.

3. Wegwerfwindel nach Anspruch 1 oder 2, wobei die zweite obere Lage (7) aus einem Faservlies besteht.

4. Verfahren zum Herstellen einer Wegwerfwindel nach einem der Ansprüche 1 bis 3, **dadurch gekennzeichnet, daß** die zweite flüssigkeitsdurchlässige Lage in in Längsrichtung und in Querrichtung gedehntem Zustand an der Windelgrundstruktur befestigt wird.

## Claims

1. A disposable diaper comprising a first liquid-permeable topsheet (1), a liquid-impermeable backsheet (2) and a liquid-absorbent panel (3) sandwiched by these sheets to form a basic diaper structure and additionally comprising a second liquid-permeable topsheet (7) laid over said first liquid-permeable topsheet (1) and bonded at its longitudinally opposite ends to said basic diaper structure and into which an aperture is formed, said second liquid-permeable topsheet (7) consisting of a sheet (7), being stretchable both in length and width and being bonded to the diaper structure when it is stretched in longitudinal direction,
**characterized in that**
said second liquid-permeable topsheet (7) also is being stretched in transverse direction and being affixed only at the afore mentioned locations and being provided at locations corresponding to the wearer's urinary organs and anus with openings being independent from each other.

2. A disposable diaper according to Claim 1, wherein said second topsheet has an elongating stress adjusted to 50 to 500g/inch and said absorbent panel (3) has a bending resistance adjusted to 5 to 20g·cm as measured according to the Taber method.

3. A disposable diaper according to Claim 1 or 2, wherein said second topsheet (7) is made of nonwoven fabric.

4. Method for manufacturing a disposable diaper according to one of the Claims 1 to 3, **characterized in that** the second liquid permeable layer (7) is affixed to the basic diaper structure in a condition being stretched in longitudinal and in transverse direction.

## Revendications

1. Couche jetable comportant une première épaisseur supérieure perméable aux liquides (1), une épaisseur inférieure imperméable aux liquides (2) et un corps d'absorption des liquides (3), qui est logé à la façon d'un sandwich entre lesdites épaisseurs, ces épaisseurs formant une structure de base de la couche, et comportant une épaisseur supérieure perméable aux liquides supplémentaire (7) disposée au-dessus de la première épaisseur supérieure perméable aux liquides (1), fixée au niveau de ses extrémités situées face à face en direction longitudinale à ladite structure de la couche et dans laquelle est configurée une ouverture, la deuxième épaisseur supérieure perméable aux liquides (7) se composant d'une épaisseur extensible (7) aussi bien en direction longitudinale qu'en direction transversale et étant fixée dans un état d'extension en direction longitudinale à ladite structure de la couche,
**caractérisée en ce que**
la deuxième épaisseur perméable aux liquides (7) est fixée uniquement au niveau des desdits endroits également dans un état d'extension en direction transversale et est pourvue d'ouvertures indépendantes l'une par rapport à l'autre aux endroits correspondant aux organes urinaires et à l'anus d'un porteur.

2. Couche jetable selon la revendication 1, dans laquelle la deuxième épaisseur supérieure présente un allongement qui est réglé à 50 à 500 g/pouce et dans laquelle le corps d'absorption (3) présente une résistance à la flexion qui est réglée à 5 à 20 g•cm, les mesures étant effectuées selon la méthode Taber.

3. Couche jetable selon la revendication 1 ou 2, dans laquelle la deuxième épaisseur supérieure (7) se compose d'un non-tissé.

4. Procédé de fabrication d'une couche jetable selon l'une des revendications 1 à 3, **caractérisé en ce que** la deuxième épaisseur perméable aux liquides est fixée à la structure de base de la couche dans un état d'extension en direction longitudinale et en direction transversale.
